# EUROPEAN PATENT APPLICATION

(11) **EP 4 707 275 A1**
(43) Date of publication of application: **11.03.2026**
(21) Application number: 24797473.6
(22) Date of filing: 26.04.2024
(51) Int. Cl.: C07D 401/12, A61K 31/517, A61K 31/496, A61K 45/06, A61P 43/00, A61P 11/00, C07D 471/04, A23L 33/10

(54) **NOVEL COMPOUND COMPRISING N-METHYLPIPERAZINE ETHANOL CARBAMATE STRUCTURE AND USE THEREOF**

(30) Priority: 28.04.2023 KR 20230056515
(71) Applicant: Korea Institute of Radiological & Medical Sciences, Seoul 01812 (KR)
(72) Inventor: LEE, Yoon-Jin, Seoul 07997 (KR); KIM, Jihee, Seoul 03763 (KR); NAM, Jae Kyung, Seoul 01658 (KR); RYU, Hyung Chu, Hwaseong-si, Gyeonggi-do 18526 (KR)
(74) Representative: Mewburn Ellis LLP
(86) International application number: PCT/KR2024/005689
(87) International publication number: WO 2024/225819

(57) **Abstract**

The present invention relates to a novel compound comprising an N-methylpiperazine ethanol carbamate structure and use thereof.

## Description

### [Technical Field]

The present invention relates to a novel compound comprising an N-methylpiperazine ethanol carbamate structure and use thereof.

### [Background Art]

Pulmonary fibrosis refers to a condition in which fibrous connective tissue proliferates in the lungs, thereby destroying the normal lung structure and resulting in the hardening and deterioration of lung tissue.

In particular, idiopathic pulmonary fibrosis (IPF) is a disease in which chronic inflammatory cells infiltrate the alveolar walls, causing various changes that harden the lungs, induce severe structural alterations in lung tissue, and gradually deteriorate pulmonary function. To date, no effective therapeutic method exists.

In addition, radiotherapy is often used to treat patients with non-small cell lung cancer (NSCLC) who are not eligible for surgical resection, but it often causes radiation-induced pulmonary fibrosis (RIPF).

To date, Ofev (Boehringer Ingelheim) containing nintedanib as an active ingredient is known to attenuate the decline in lung function to some extent. However, there has been a growing need to develop more effective therapeutic agents.

### [Disclosure]

### [Technical Problem]

As a result of continuous efforts to develop a more effective drug for the prevention or treatment of pulmonary fibrosis, the present inventors have confirmed the preventive or therapeutic effect of a novel compound comprising an N-methylpiperazine ethanol carbamate structure on pulmonary fibrosis, thereby completing the present invention.

### [Technical Solution]

An object of the present invention is to provide a compound represented by following Formula 1, or a pharmaceutically acceptable salt or solvate thereof: 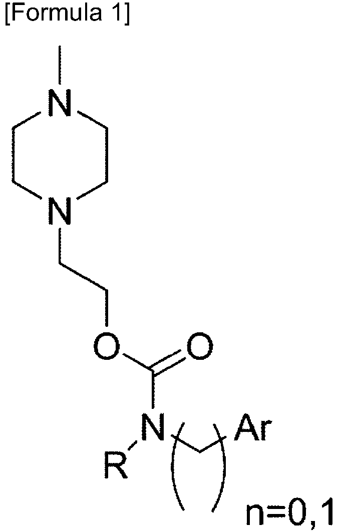
wherein in Formula 1 above, Ar is aryl or heteroaryl, substituted with trifluoromethyl (CF3) or halogen,
and R is represented by following Formula 2 or Formula 3:

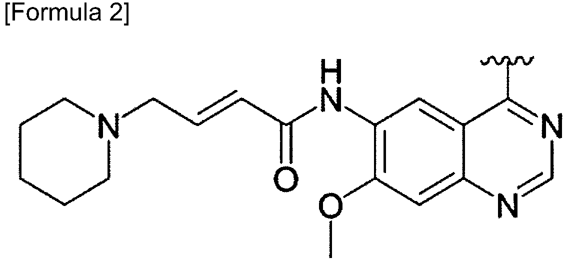

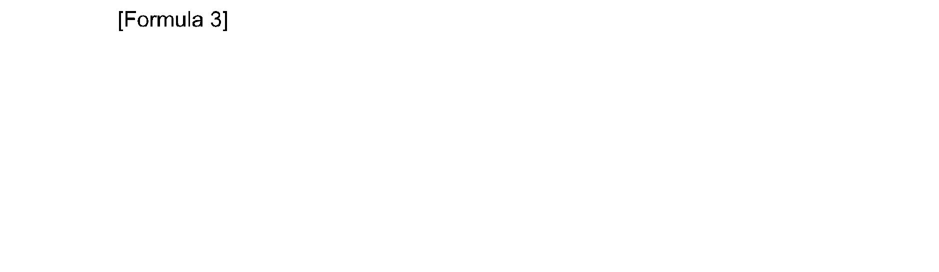

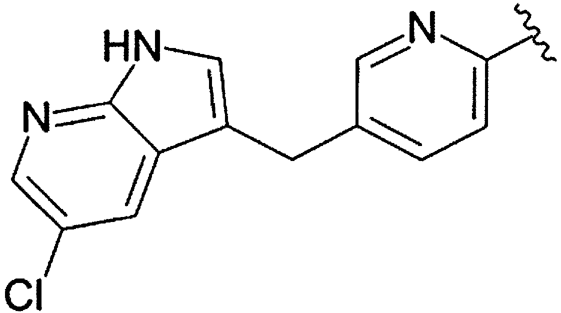

In one embodiment, Ar in Formula 1 above is 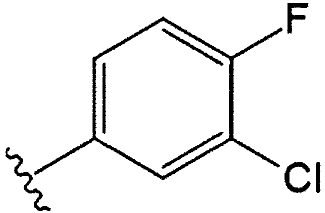 or

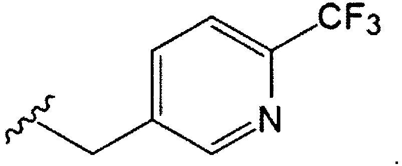

In any one embodiment of the foregoing embodiment, the compound represented by Formula 1 is a compound represented by Formula 4 or Formula 5.

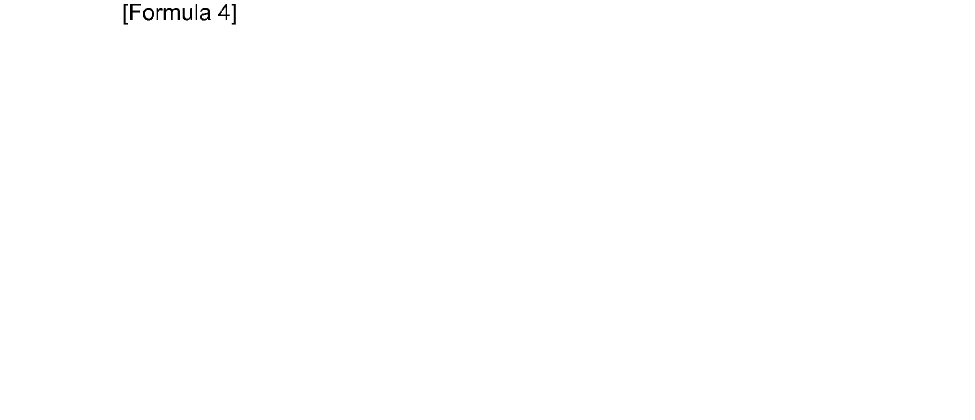

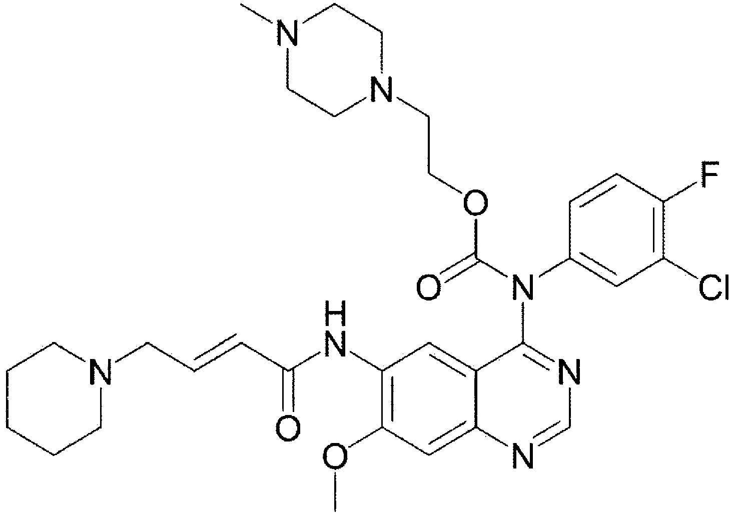

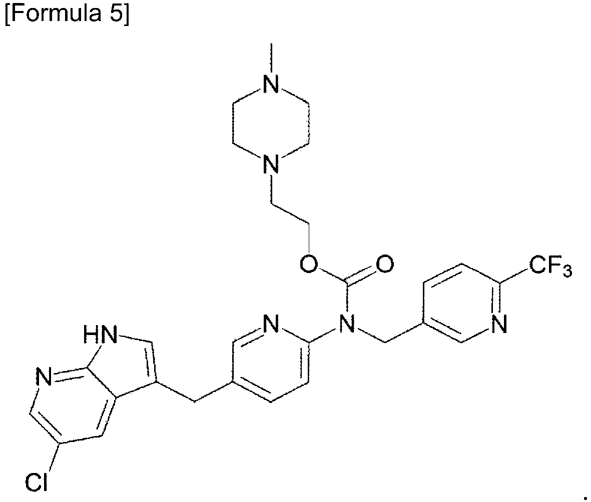

Another object of the present invention is to provide a composition comprising the compound represented by Formula 1 or a pharmaceutically acceptable salt or solvate thereof, and a pharmaceutically acceptable carrier.

Still another object of the present invention is to provide a pharmaceutical composition for preventing or treating pulmonary fibrosis, comprising the compound represented by Formula 1 or a pharmaceutically acceptable salt or solvate thereof:
Still another object of the present invention is to provide a method for preventing or treating pulmonary fibrosis, comprising administering the pharmaceutical composition to a subject.

Still another object of the present invention is to provide a food composition for preventing or improving pulmonary fibrosis, comprising the compound represented by Formula 1 or a food-acceptable salt or solvate thereof.

### [Advantageous Effects]

The novel compound of the present invention can be usefully employed for the prevention or treatment of pulmonary fibrosis.

### [Brief Description of the Drawings]

FIG. 1 shows the results of MS analysis (FIG. 1a) and H-NMR analysis (FIGS. 1b to 1d) of synthesized DCTP.
FIG. 2 confirms the effect of inhibiting idiopathic pulmonary fibrosis (IPF), confirmed by Micro-CT imaging, after administration of nintedanib and/or DCTP to mice in which idiopathic pulmonary fibrosis was induced by administration of bleomycin.
FIG. 3 shows a graph illustrating the degree of inflammation and fibrosis, confirmed by H&E and Masson's Trichrome staining, after administration of nintedanib and/or DCTP to mice in which idiopathic pulmonary fibrosis was induced by administration of bleomycin.
FIG. 4 shows the effect of inhibiting radiation-induced pulmonary fibrosis (RIPF), confirmed by Micro-CT imaging, after administration of nintedanib and/or DCTP to mice in which pulmonary fibrosis was induced by radiation exposure.
FIG. 5 shows a graph illustrating the degree of inflammation and fibrosis, confirmed by H&E and Masson's Trichrome staining, after administration of nintedanib and/or DCTP to mice in which idiopathic pulmonary fibrosis was induced by radiation exposure.
FIG. 6 shows the results of MS analysis (FIG. 6a) and H-NMR analysis (FIG. 6b) of synthesized PXDP.
FIG. 7 shows the effect of inhibiting idiopathic pulmonary fibrosis, confirmed by Micro-CT imaging, after administration of nintedanib and/or PXDP to mice in which idiopathic pulmonary fibrosis was induced by administration of bleomycin.
FIG. 8 shows a graph illustrating the degree of inflammation and fibrosis, confirmed by H&E and Masson's Trichrome staining, after administration of nintedanib and/or PXDP to mice in which idiopathic pulmonary fibrosis was induced by administration of bleomycin.
FIG. 9 shows the results of confirming the survival rate after administration of nintedanib and/or PXDP to mice in which idiopathic pulmonary fibrosis was induced by administration of bleomycin.
FIG. 10 shows the effect of inhibiting radiation-induced pulmonary fibrosis (RIPF), confirmed by Micro-CT imaging, after administration of nintedanib and/or PXDP to mice in which pulmonary fibrosis was induced by radiation exposure.
FIG. 11 shows a graph illustrating the degree of inflammation and fibrosis, confirmed by H&E and Masson's Trichrome staining, after administration of nintedanib and/or PXDP to mice in which idiopathic pulmonary fibrosis was induced by radiation exposure.

### [Detailed Description of Preferred Embodiments]

The present invention will be described in detail as follows. Meanwhile, each description and embodiment described herein may be applied to other descriptions and embodiments, respectively. That is, all combinations of various elements described herein fall within the scope of the present invention. Further, the scope of the present invention is not limited by the specific description described below.

Furthermore, those skilled in the art may recognize or identify numerous equivalents to the specific embodiments of the present invention described herein by using routine experimentation. Moreover, such equivalents are intended to be included within the present invention.

Additionally, a number of papers and patent documents have been cited throughout the present specification. The content of the cited papers and patent documents is incorporated herein by reference in their entirety to describe the level of the technical field to which the present invention belongs and the contents of the present invention more clearly.

One aspect of the present invention provides a compound comprising an N-methylpiperazine ethanol carbamate structure, which is represented by following Formula 1, or a pharmaceutically acceptable salt or solvate thereof: 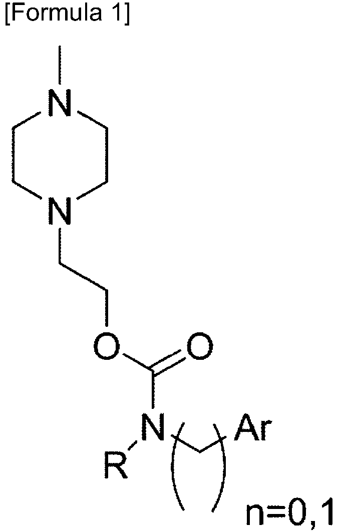
wherein in Formula 1 above, Ar is aryl or heteroaryl, substituted with trifluoromethyl (CF3) or halogen,
and R is represented by following Formula 2 or Formula 3:

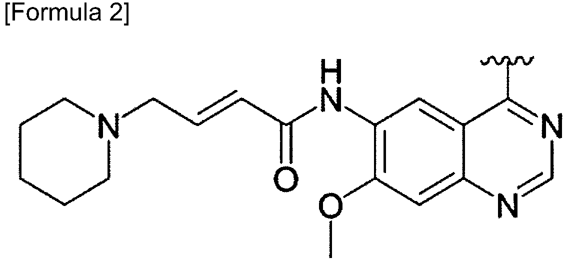

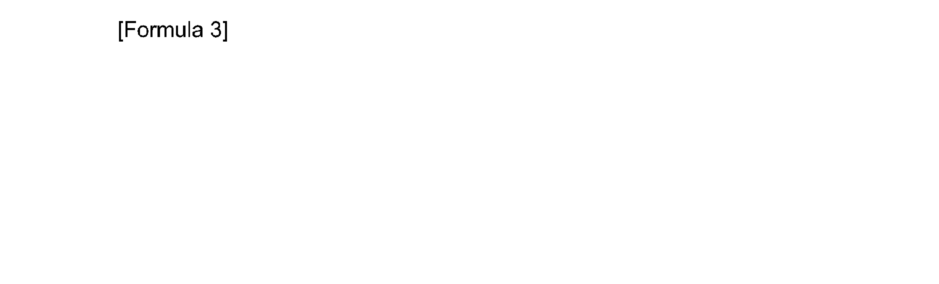

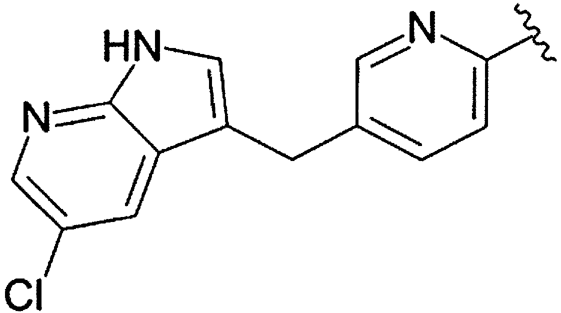

For example, the halogen may be Cl or F.

For example, Ar in Formula 1 above may be 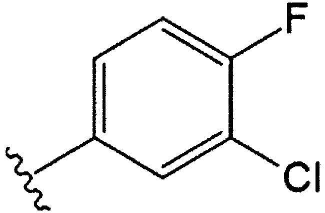 or

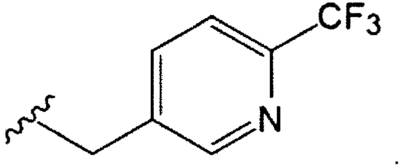

For example, the compound represented by Formula 1 of the present invention may have the structure of Formula 4:

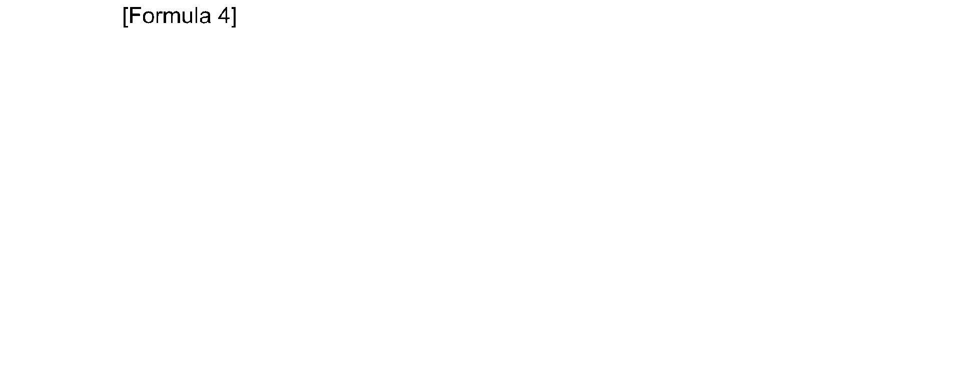

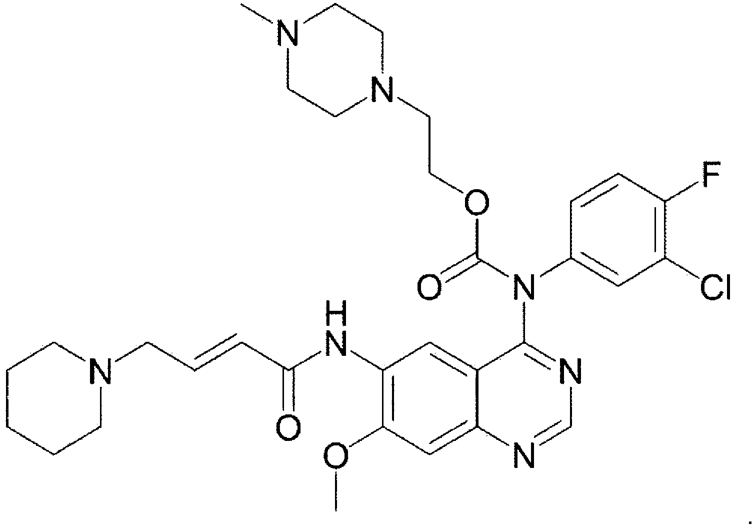

The compound of Formula 4 is a compound referred to as 2-(4-methylpiperazin-1-yl)ethyl (E)-(3-chloro-4-fluorophenyl)(7-methoxy-6-(4-(piperidin-1-yl)but-2-enamido)quinazolin-4-yl)carbamate, and may be referred to as "DCTP" in the present invention.

For example, the compound represented by Formula 1 of the present invention may have the structure of Formula 5:

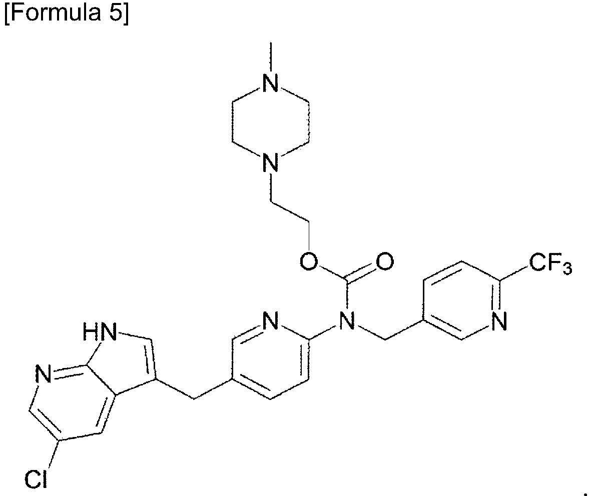

The compound of Formula 5 is a compound referred to as 2-(4-methylpiperazin-1-yl)ethyl (5-((5-chloro-1H-pyrrolo[2,3-b]pyridin-3-yl)methyl)pyridin-2-yl)((6-(trifluoromethyl)pyridin-3-yl)methyl)carbamate, and may be referred to as "PXDP" in the present invention.

The compound provided herein encompasses a compound represented by a specific formula as well as its clathrates, hydrates, solvates, or polymorphs. Additionally, the term "compound of the present invention" includes pharmaceutically acceptable salts thereof, even when the pharmaceutically acceptable salts are not explicitly mentioned. In one embodiment, the compound of the present invention may exist as a stereoisomerically pure compound (for example, substantially free of other stereoisomers (for example, at least 85% ee, at least 90% ee, at least 95% ee, at least 97% ee, or at least 99% ee)), but is not limited thereto.

The term "hydrate" refers to a compound of the present invention that includes a stoichiometric or non-stoichiometric amount of water bound by non-covalent intermolecular forces, or a pharmaceutically acceptable salt thereof.

The term "clathrate" refers to a compound of the present invention in the form of a crystal lattice that includes spaces (for example, channels) in which guest molecules (for example, solvent or water) are trapped, or a salt thereof.

The term "pharmaceutically acceptable" means that it is suitable for use as a pharmaceutical preparation, and may be used, within the scope of sound medical judgment, to refer to a compound, substance, composition, and/or form of administration that is suitable for contact with human or animal tissues without undue toxicity, irritation, allergic reaction, or other problems or complications and that has a reasonable benefit-to-risk ratio.

The term "pharmaceutically acceptable salt" in the present invention refers to a derivative of a known compound in which a parent compound is modified by forming an acid or base salt thereof. Examples of the pharmaceutically acceptable salt include mineral or organic acid salts of basic residues such as amines, and alkali or organic salts of acidic residues such as carboxylic acids, but are not limited thereto. Examples of the pharmaceutically acceptable salt of the present invention include commonly used salts in the pharmaceutical field, such as hydrochloride, hydrobromide, hydroiodide, hydrofluoride, sulfate, sulfonate, citrate, camphorate, maleate, acetate, lactate, nicotinate, nitrate, succinate, phosphate, malonate, malate, salicylate, phenylacetate, stearate, formate, fumarate, urea, sodium, potassium, calcium, magnesium, zinc, lithium, cinnamate, methylamino, methanesulfonate, picrate, p-toluenesulfonate, naphthalenesulfonate, tartrate, triethylamino, dimethylamino, and tris(hydroxymethyl)aminomethane salts, but are not limited thereto.

The pharmaceutically acceptable salt of the compound of the present invention may be synthesized from a parent compound comprising a basic or acidic moiety by using a conventional chemical method. Generally, such a salt may be prepared by reacting the compound in free acid or base form with a sufficient amount of a suitable base or acid in water or in an organic diluent such as ether, ethyl acetate, ethanol, isopropanol, or acetonitrile, or a mixture thereof.

The term "solvate" or "pharmaceutically acceptable solvate" refers to a solvate formed from the association of one or more solvent molecules with the compound. The term "solvate" includes hydrates (for example, hemihydrates, monohydrates, dihydrates, trihydrates, or tetrahydrates).

Another aspect of the present invention provides a pharmaceutical composition for preventing or treating pulmonary fibrosis, comprising the compound represented by Formula 1 or a pharmaceutically acceptable salt or solvate thereof.

The term "pulmonary fibrosis" refers to a respiratory disease in which lung tissue becomes hardened, causing severe respiratory dysfunction. Pulmonary fibrosis may occur due to various causes such as radiation, tuberculosis, syphilis, pneumoconiosis, or viral infections, and may also occur for unknown reasons. All such pulmonary fibrosis falls within the scope of pulmonary fibrosis of the present invention.

In one embodiment of the present invention, the pulmonary fibrosis may be selected from pulmonary fibrosis caused by radiation exposure and idiopathic pulmonary fibrosis.

The term "idiopathic pulmonary fibrosis" (IPF) of the present invention refers to a disease of unknown cause among interstitial lung diseases, wherein repeated inflammation resulting from alveolar injury induces fibrosis and leads to respiratory failure in patients.

In one embodiment of the present invention, pulmonary fibrosis may be a side effect of radiotherapy in which normal tissues are also exposed to radiation during radiotherapy for cancer treatment, or a side effect of chemotherapy used for cancer treatment.

Examples of radiotherapy or chemotherapy for cancer that may induce pulmonary fibrosis may include the treatment of breast cancer, lung cancer, bone cancer, pancreatic cancer, skin cancer, oral cancer, oropharyngeal cancer, uterine cancer, ovarian cancer, rectal cancer, stomach cancer, endometrial carcinoma, cervical carcinoma, vaginal carcinoma, small intestine cancer, thyroid cancer, parathyroid cancer, prostate cancer, chronic or acute leukemia, lymphocytic lymphoma, bladder cancer, kidney cancer, liver cancer, colon cancer, or brain tumors, but are not limited thereto.

In one embodiment, the pharmaceutical composition of the present invention may be administered before or after radiation exposure.

The term "treatment" refers to any activity that improves or beneficially changes a symptom of fibrosis by administering the pharmaceutical composition, and the term "prevention" refers to any activity that inhibits or delays the onset of fibrosis by administering the pharmaceutical composition.

The pharmaceutical composition according to the present invention may be prepared by a conventional method used in the pharmaceutical field. The pharmaceutical composition may be formulated with a suitable pharmaceutically acceptable carrier depending on the dosage form, and may be prepared by further comprising an excipient, diluent, dispersant, emulsifier, buffer, stabilizer, binder, disintegrant, solvent, or the like, if necessary. The suitable carrier or the like does not impair the activity or properties of the compound according to the present invention, and may be selected according to the form of administration and dosage form.

The pharmaceutical composition of the present invention may further comprise a suitable carrier, excipient, or diluent that is commonly used in the preparation of a pharmaceutical composition. The composition comprising a pharmaceutically acceptable carrier may be in various oral or parenteral dosage forms. When formulated, the composition may be prepared using a commonly used diluent or excipient such as a filler, extender, binder, wetting agent, disintegrant, or surfactant. Solid preparations for oral administration may include tablets, pills, powders, granules, capsules, *etc.*, and such solid preparations may be formulated by mixing, into the composition, at least one or more excipients, such as starch, calcium carbonate, sucrose, lactose, and gelatin. Further, in addition to simple excipients, lubricants such as magnesium stearate and talc may be used. Examples of liquid preparations for oral administration may include suspensions, liquids for internal use, emulsions, syrups, *etc.*, and in addition to commonly used simple diluents including water and liquid paraffin, various excipients such as wetting agents, sweeteners, flavoring agents, and preservatives may be included. Preparations for parenteral administration may include sterilized aqueous solutions, non-aqueous solvents, suspensions, emulsions, freeze-dried preparations, and suppositories. As non-aqueous solvents and suspending agents, propylene glycol, polyethylene glycol, vegetable oils such as olive oil, injectable esters such as ethyl oleate, *etc.* may be used. As bases for suppositories, Witepsol, Macrogol, Tween 61, cocoa butter, lauric acid, glycerogelatin, *etc.* may be used.

In addition, the pharmaceutical composition of the present invention may, without being limited thereto, be in a dosage form selected from the group consisting of tablets, pills, powders, granules, capsules, suspensions, liquids for internal use, emulsions, syrups, sterile aqueous solutions, non-aqueous solutions, suspensions, emulsions, lyophilized preparations, and suppositories.

The pharmaceutical composition according to the present invention may be administered in a pharmaceutically effective amount.

The term "pharmaceutically effective amount" refers to an amount that is sufficient to inhibit or relieve increased vascular permeability with a reasonable benefit-risk ratio applicable to medical use. The effective dose level may be determined based on factors including the subject type, severity, age, sex, drug activity, sensitivity to drugs, administration time, route of administration and elimination rate, treatment duration, and combination or concurrent use of drugs, as well as other well-known factors in the medical field.

The effective dose level of the pharmaceutical composition may be determined based on factors including the target use, age, sex, weight, and health conditions of the patient, type and severity of the disease, drug activity, sensitivity to drugs, administration method, administration time, route of administration and elimination rate, treatment duration, and combination or concurrent use of drugs, as well as other well-known factors in the medical field. For example, although not consistent, it may generally be administered once or several times daily in an amount of 0.001 mg/kg to 100 mg/kg, for example, 0.01 mg/kg to 10 mg/kg. However, the administered dose does not limit the scope of the present invention by any means.

The pharmaceutical composition may be appropriately administered to a subject based on the conventional method, route of administration, and administered dose used in the art, depending on the purpose or need. Examples of the route of administration include oral, parenteral, subcutaneous, intraperitoneal, intrapulmonary, and intranasal administration. Parenteral injection comprises intramuscular, intravenous, intraarterial, intraperitoneal, or subcutaneous administration. Additionally, suitable dose and administration frequency may be selected according to a known method in the art. The actual amount and administration frequency of the pharmaceutical composition of the present invention to be administered may be appropriately determined based on various factors such as the type of symptom to be treated, route of administration, sex, health conditions, diet, age, and body weight of the subject, and severity of the disease.

The term "administration" refers to the introduction of the pharmaceutical composition of the present invention to a subject using a suitable method, and the route of administration includes various oral and parenteral routes, as long as it allows the composition to reach the target tissue.

The pharmaceutical composition may be administered to any animal in which pulmonary fibrosis may occur, and the animal may include, for example, livestock such as cattle, pigs, horses, and dogs, in addition to humans and primates. In certain embodiments, the animal may be an animal excluding humans.

The pharmaceutical composition may be administered through a suitable route of administration for the dosage form, and may be administered using various oral or parenteral routes, as long as it allows the composition to reach the target tissue. The administration method may, without being limited thereto, be administered using a conventional method including, for example, oral, rectal or intravenous, intramuscular, transdermal administration, inhalation, intrauterine epidural, or intracerebroventricular injection.

The pharmaceutical composition of the present invention may be administered in combination with another composition for preventing or treating pulmonary fibrosis, in addition to the composition comprising the compound represented by Formula 1 according to the present invention or a pharmaceutically acceptable salt or solvate as an active ingredient.

Examples of compositions that can be administered in combination with the pharmaceutical composition of the present invention include compositions comprising any one or more ingredients selected from pirfenidone, nintedanib, steroids, azathioprine, cyclophosphamide, and a pharmaceutically acceptable salt or solvate thereof.

In one embodiment, the pharmaceutical composition of the present invention is administered in combination with a pharmaceutical composition comprising nintedanib as an active ingredient.

As used herein, the term "combination administration", "co-administration", or "administration in combination" means that a composition comprising the compound represented by following Formula 1, or a pharmaceutically acceptable salt or solvate thereof as an active ingredient is administered to a subject, together with another antifibrotic agent. This is not limited to the simultaneous administration of the co-administered drugs, and may be in a dosage form in which the compound of the present invention and another antifibrotic agent act together in a subject such that each substance can perform its inherent function at an equivalent or higher level. Accordingly, as used herein, when the term "combination" is used, it refers to simultaneous, separate, sequential, or reverse administration, and is to be construed as being unrestricted in order. When the administration is sequential, reverse, or separate, the order of administration is not particularly limited, but the interval between the administration of the second ingredient should not lead to the loss of the beneficial effect of the combination administration.

In the present invention, (i) the composition comprising the compound represented by Formula 1 or a pharmaceutically acceptable salt or solvate thereof, and (ii) the composition comprising nintedanib or a pharmaceutically acceptable salt or solvate thereof may be administered in a form as described below, but are not limited thereto:
a) administration of a mixture in which (i) the compound represented by Formula 1, or a pharmaceutically acceptable salt or solvate thereof, and (ii) nintedanib or a pharmaceutically acceptable salt or solvate thereof are mixed; or
b) administration of (i) the compound represented by Formula 1, or a pharmaceutically acceptable salt or solvate thereof, and (ii) nintedanib or a pharmaceutically acceptable salt or solvate thereof in separate forms, but not limited thereto.

When (i) the compound represented by Formula 1, or a pharmaceutically acceptable salt or solvate thereof, and (ii) nintedanib or a pharmaceutically acceptable salt or solvate thereof are administered in separate forms, the (i) and (ii) may be formulated into separate formulations and administered simultaneously, separately, sequentially, or in reverse order.

The therapeutically effective dose of each active ingredient used in the combination administration may vary depending on the particular compound or pharmaceutical composition used, the mode of administration, the symptom to be treated, the severity of the symptom, and the species, body weight, sex, dietary conditions, and age of the warm-blooded animal. Accordingly, the administration regimen using the compounds of the present invention is selected depending on various factors, including the route of administration and the renal and hepatic functions of the patient. A surgeon, clinician, or veterinarian skilled in the art can readily determine and prescribe an effective amount of a drug required to prevent, counteract, or arrest the progression of symptoms. The optimal precision in achieving a drug concentration within the range that provides efficacy without toxicity requires a regimen based on the pharmacokinetics of drug availability at the targeted site. It includes taking into consideration the distribution, equilibrium, and elimination of the drug. Accordingly, the dosage regimen, that is, the administration level and frequency of any individual component of the formulation of the present invention as described below, may be adjusted to provide an optimal therapeutic response.

Still another aspect of the present invention provides a combination comprising: the compound of Formula 1 or a pharmaceutically acceptable salt or solvate thereof; and nintedanib or a pharmaceutically acceptable salt thereof.

As used herein, the term "combination" refers to the use for combination administration of the compound of Formula 1 or a pharmaceutically acceptable salt or solvate thereof, and nintedanib or a pharmaceutically acceptable salt or solvate thereof, and may be understood as having the same meaning as "combination use". This also encompasses, but is not limited to, a pharmaceutical composition and a pharmaceutical kit characterized by the combination of the compound of Formula 1 or a pharmaceutically acceptable salt or solvate thereof, and nintedanib or a pharmaceutically acceptable salt or solvate thereof.

As used herein, the term "kit" may comprise the combination or composition according to the present invention for the combination administration of a compound of Formula 1 or a pharmaceutically acceptable salt or solvate thereof, and nintedanib and a pharmaceutically acceptable salt or solvent thereof. Specifically, the kit of the present invention may comprise the compound of Formula 1 or a pharmaceutically acceptable salt or solvate thereof, and nintedanib or a pharmaceutically acceptable salt or solvate thereof as a single formulation, or comprise the compound of Formula 1 or a pharmaceutically acceptable salt or solvate thereof, and nintedanib or a pharmaceutically acceptable salt or solvate thereof as separate formulations. The kit may further comprise a substance necessary for the combination administration of the two substances. However, the kit is not limited thereto.

Still another aspect of the present invention provides a method for preventing or treating pulmonary fibrosis comprising administering, to a subject, a pharmaceutical composition comprising the compound represented by Formula 1 or a food-acceptable salt or solvate thereof.

Still another aspect of the present invention provides use of the compound represented by Formula 1 or a food-acceptable salt or solvate thereof for use in a composition for preventing or treating pulmonary fibrosis.

The compound represented by Formula 1, or a food-acceptable salt or solvate thereof, pulmonary fibrosis, prevention, and treatment are as described in other aspects above.

Still another aspect of the present invention provides a food composition for preventing or improving pulmonary fibrosis, comprising the compound represented by following Formula 1 or a food acceptable salt or solvate thereof.

As used herein, the term "improvement" refers to any activity that at least reduces a parameter associated with pulmonary fibrosis, for example, the severity of a symptom, through the administration of the composition of the present invention.

The compound represented by Formula 1, or a food-acceptable salt or solvate thereof, pulmonary fibrosis, and prevention are as described in other aspects above.

The term "food-acceptable salt" refers to a salt that can be used for food applications, and specific examples thereof include the examples of "pharmaceutically acceptable salts" described above.

The food composition of the present invention may be used as a health functional food.

The term "health functional food" refers to a food manufactured and processed using raw materials or ingredients having functionalities beneficial to the human body, and the term "functionality" refers to the intake for the purpose of obtaining beneficial effects for health use, such as regulating nutrients or exerting physiological actions on the structure and function of the human body.

The food composition of the present invention may further comprise additives commonly used in food compositions to enhance aroma, taste, appearance, *etc.* For example, the additives may include vitamins such as vitamins A, C, D, E, B1, B2, B6, and B12, niacin, biotin, folate, and pantothenic acid. Additionally, the additives may include minerals such as zinc (Zn), iron (Fe), calcium (Ca), chromium (Cr), magnesium (Mg), manganese (Mn), and copper (Cu). The additives may include amino acids such as lysine, tryptophan, cysteine, and valine. Food additives such as preservatives (e.g., potassium sorbate, sodium benzoate, salicylic acid, sodium dehydroacetate), sterilizers (*e.g.*, bleaching powder, high-strength bleaching powder, sodium hypochlorite), antioxidants (e.g., butylated hydroxyanisole (BHA), butylated hydroxytoluene (BHT)), coloring agents (e.g., tar dyes), coloring agents (e.g., sodium nitrite, sodium ascorbate), bleaching agents (e.g., sodium sulfite), seasonings (e.g., monosodium glutamate (MSG)), sweeteners (*e.g.*, dulcin, cyclamate, saccharin, sodium), flavoring agents (*e.g.*, vanillin, lactones), leavening agents (e.g., alum, D-potassium hydrogen tartrate), fortifiers, emulsifiers, thickeners (stabilizers), coating agents, gum bases, anti-foaming agents, solvents, and modifiers may be added. The additives are selected based on the type of food and may be used in an appropriate amount.

When using the food composition of the present invention as a food additive, it may be added as-is or used together with another food or food ingredients, and may be appropriately used according to conventional methods.

### [Mode for Carrying Out the Invention]

Hereinafter, the present invention will be described in more detail by way of Examples and Experimental Examples. However, these Examples and Experimental Examples are given for illustrative purposes only, and the scope of the present invention is not intended to be limited by these Examples and Experimental Examples.

### Example 1. Synthesis of 2-(4-methylpiperazin-1-yl)ethyl (5-((5-chloro-1H-pyrrolo[2,3-b]pyridin-3-yl)methyl)pyridin-2-yl)((6-(trifluoromethyl)pyridin-3-yl)methyl)carbamate (DCTP)

### Example 1-1. Synthesis of intermediate (E)-(3-chloro-4-fluorophenyl)(7-methoxy-6-(4-(piperidin-1-yl)but-2-enamido)quinazolin-4-yl)carbamic chloride

(E)-N-(4-((3-chloro-4-fluorophenyl)amino)-7-methoxyquinazolin-6-yl)-4-(piperidin-1-yl)but-2-enamide (500 mg, 1.06 mmol) was suspended in anhydrous dichloromethane (10 mL), followed by the addition of pyridine (126.2 mg, 1.59 mmol), and the mixture was stirred under a nitrogen atmosphere at 0°C to 5°C. Triphosgene (189.4 mg, 0.63 mmol) was added, and the mixture was stirred at room temperature for at least 1 hour. The reaction mixture was concentrated under reduced pressure, and the resulting residue was purified by silica gel column chromatography (10% MC:MeOH) to afford a light yellow solid (178.4 mg, 31.5%).

### Example 1-2. Synthesis of 2-(4-methylpiperazin-1-yl)ethyl (E)-(3-chloro-4-fluorophenyl)(7-methoxy-6-(4-(piperidin-1-yl)but-2-enamido)quinazolin-4-yl)carbamate

2-(4-methylpiperazin-1-yl)ethan-1-ol (44.6 mg, 0.31 mmol) was added to anhydrous dichloromethane (3.0 mL), followed by the addition of 60% sodium hydride (16.9 mg, 0.42 mmol) under a nitrogen atmosphere at 0°C to 5°C. The mixture was then stirred. The intermediate (E)-(3-chloro-4-fluorophenyl)(7-methoxy-6-(4-(piperidin-1-yl)but-2-enamido)quinazolin-4-yl)carbamic chloride obtained in Example 1-1 was added, and the mixture was stirred at room temperature for at least 1 hour. The reaction mixture was concentrated under reduced pressure, and the resulting residue was purified by silica gel column chromatography (20% MC:MeOH) to afford a yellow solid (41.8 mg, 23.2%).

Chemical Formula: C₃₂H₃₉ClFN₇O₄

Exact Mass: 639.27
LC/MS: [M+H] 640.28
¹H-NMR(DMSO-d6): δ 9.78(1H), 9.03(1H), 9.00(1H), 7.72-7.70(1H), 7.54(1H), 7.45-7.41(1H), 7.32-7.28(1H), 6.83-6.77(1H), 6.68-6.64(1H), 4.22-4.19(2H), 4.11(3H), 3.18-3.17(2H), 3.09-3.08(2H), 2.38-2.33(8H), 2.14-2.10(4H), 2.08(3H), 1.55-1.49(4H), 1.40-1.39(2H)

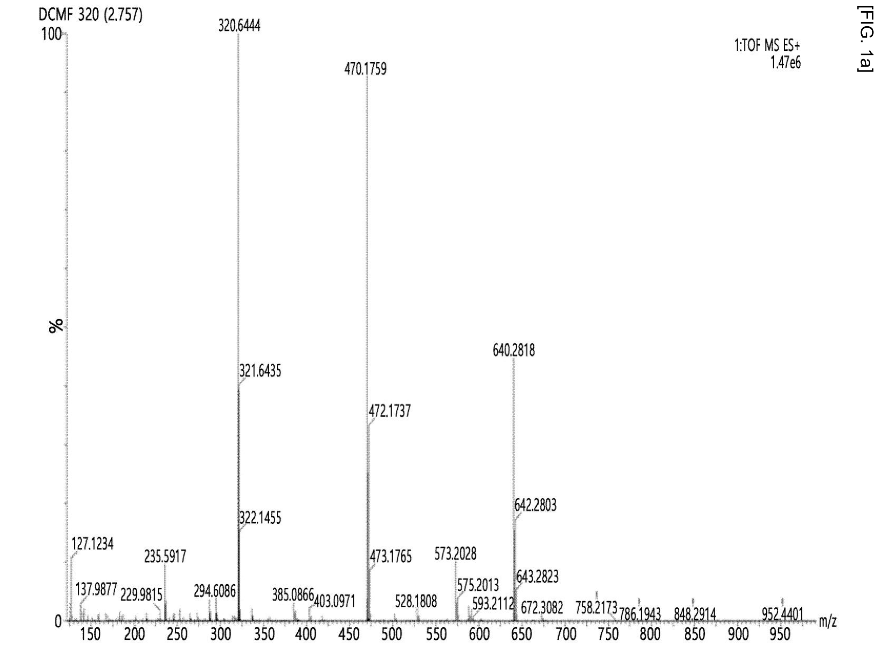

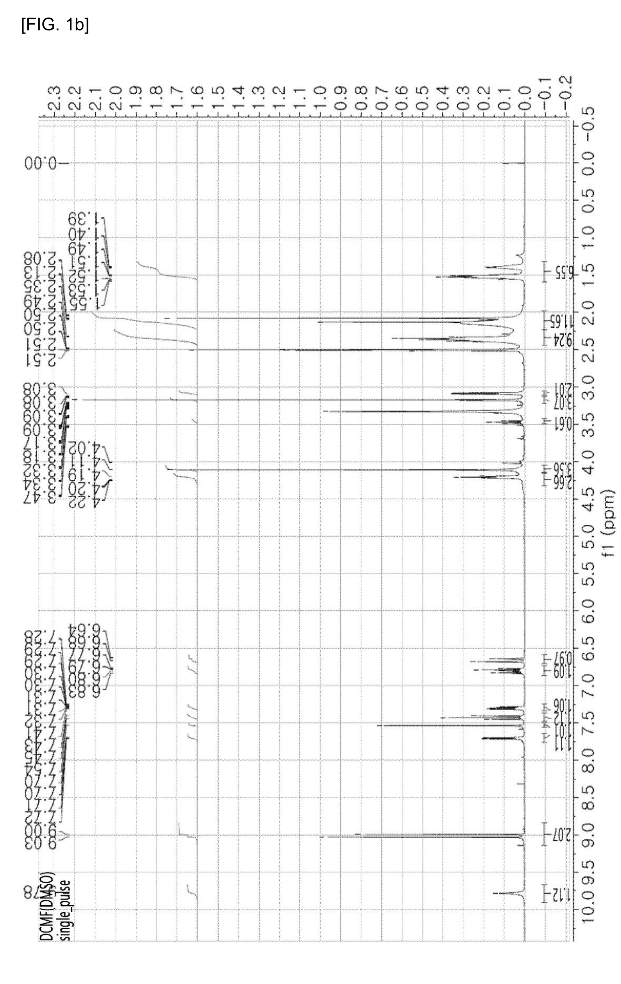

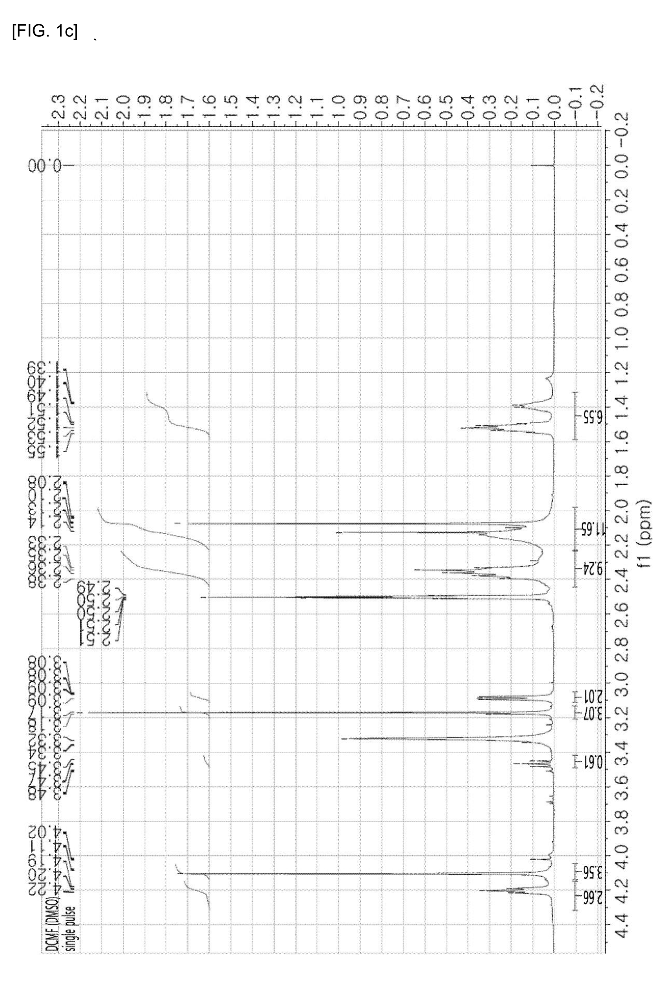

MS and NMR data are shown in FIG. 1, and the obtained compound, 2-(4-methylpiperazin-1-yl)ethyl (E)-(3-chloro-4-fluorophenyl)(7-methoxy-6-(4-(piperidin-1-yl)but-2-enamido)quinazolin-4-yl)carbamate (hereinafter referred to as "DCTP"), was used in Examples 2 and 3 below.

### Example 2. Confirmation of inhibitory effect on idiopathic pulmonary fibrosis induced by bleomycin

Bleomycin sulfate was administered at a dose of 1.5 U/kg to six-week-old male C57BL/6 mice, and the mice were observed for pulmonary fibrosis (10 mice per group). Each drug was administered starting 14 days after the administration of bleomycin. The drug was orally administered at a dose of 20 mg/kg once daily for two weeks (a total of 13 administrations). The control drug, nintedanib, was administered at a dose of 60 mg/kg, and the co-administration group was orally administered nintedanib (60 mg/kg) together with DCTP (20 mg/kg) once daily for two weeks (a total of 13 administrations).

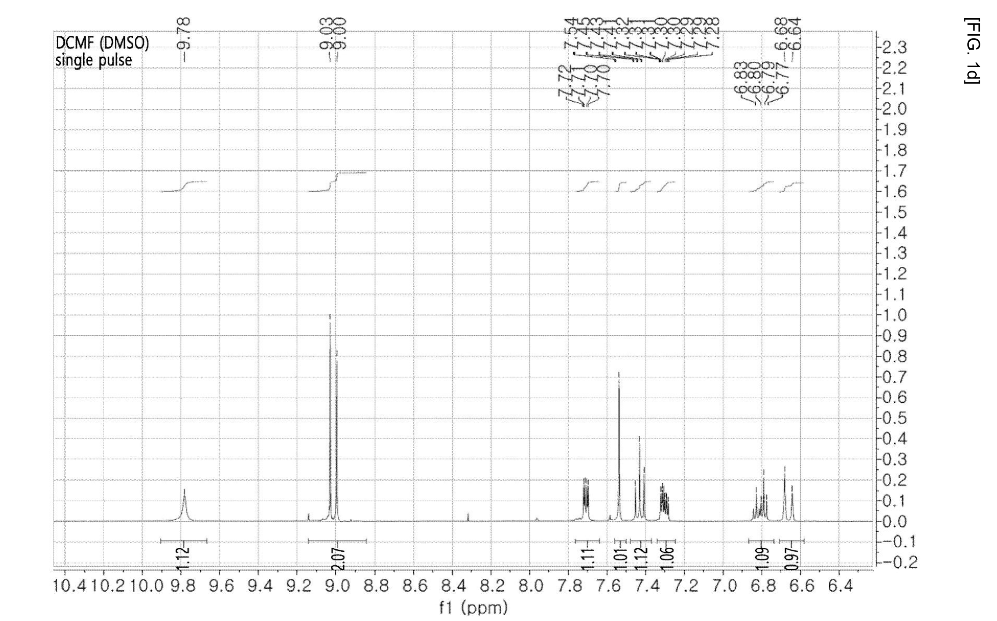

The results of Micro-CT imaging performed 4 weeks after bleomycin administration are shown in FIG. 2.

In the untreated group, progression of fibrosis was observed 4 weeks after bleomycin administration, whereas in the group treated with nintedanib, which is a known antifibrotic agent, the Micro-CT imaging results showed a reduction in the degree of fibrosis. In addition, it was found that the group treated with the combination of nintedanib and DCTP exhibited a greater fibrotic effect.

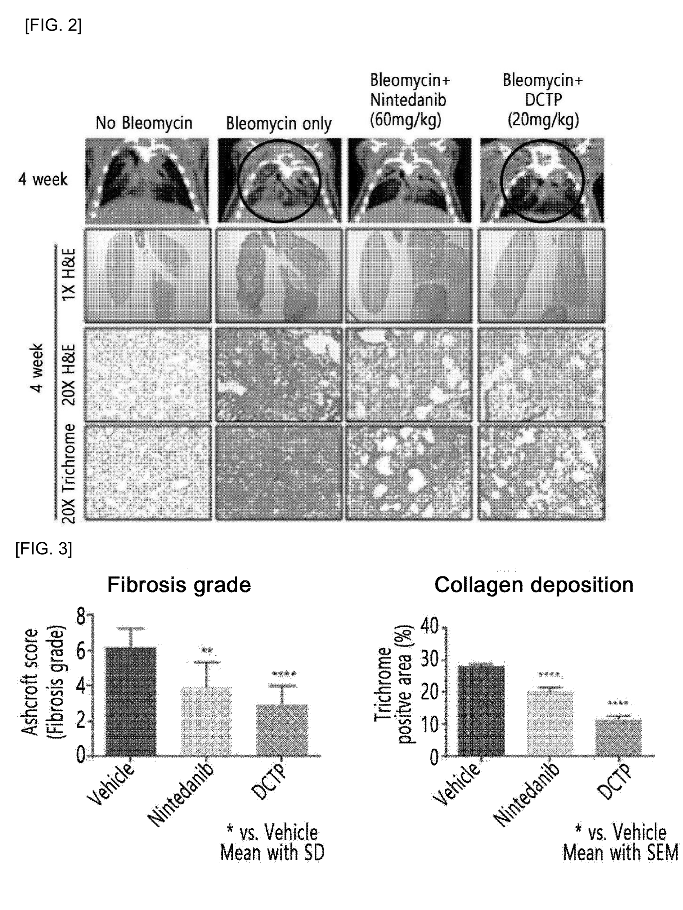

The results of H&E and Masson's Trichrome staining performed to evaluate the degree of inflammation and fibrosis are shown in FIG. 3.

Compared to the untreated group, the group treated with nintedanib showed alleviation of pulmonary fibrosis and inflammation. In particular, the group treated with prof nintedanib and DCTP showed a greater reduction in the degree of fibrosis and inflammation.

### Example 3. Confirmation of inhibitory effect on radiation-induced pulmonary fibrosis

One hour before stereotactic body radiation therapy (SBRT; 90 Gy, 4 mm collimator), DCTP and nintedanib, an FDA-approved drug known to inhibit pulmonary fibrosis, were orally administered to seven-week-old male C57BL/6 mice. The degree of pulmonary fibrosis and the inhibitory effect on radiation-induced lung injury were observed two weeks after radiation exposure.

The DCTP drug was administered at a dose of 30 mg/kg, and nintedanib was administered once daily (o.d.) at a dose of 60 mg/kg.

The results of Micro-CT imaging performed 2 weeks after radiation exposure are shown in FIG. 4.

In the untreated group, progression of fibrosis was observed 2 weeks after radiation exposure, whereas in the group treated with nintedanib, which is a known antifibrotic agent, the Micro-CT imaging results showed reduction in the degree of fibrosis.

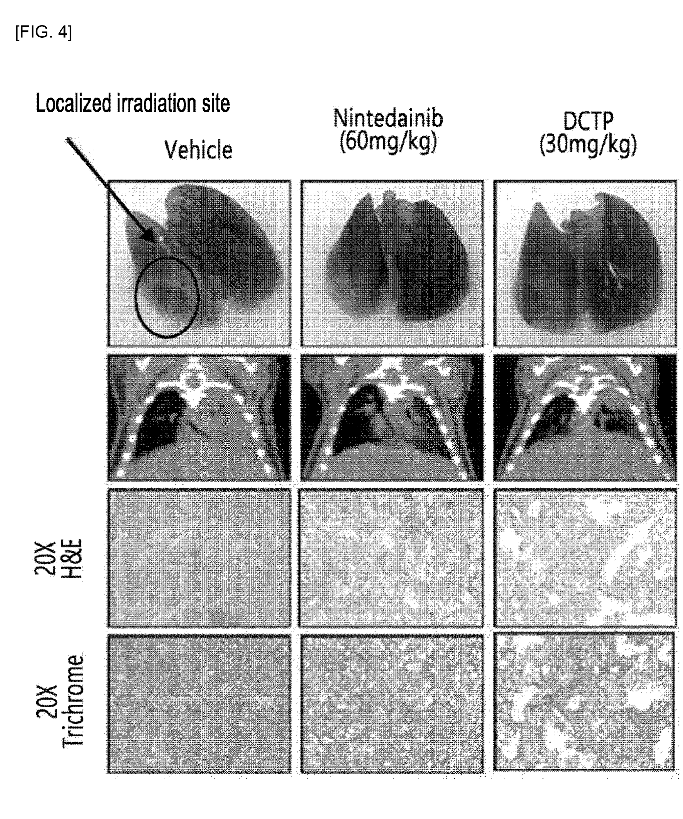

In addition, to analyze the degree of inflammatory response and the degree of fibrosis two weeks after radiation exposure, H&E staining and Masson's Trichrome staining were performed, and the results are shown in FIG. 5.

The results of H&E and Masson's Trichrome staining that were performed to confirm the degree of inflammation and the degree of fibrosis confirmed that, compared to the untreated group, both the nintedanib-treated group and the DCTP-treated group exhibited a reduction in fibrosis and inflammation in the lungs (FIG.5).

### Example 4. Synthesis of (2-(4-methylpiperazin-1-yl)ethyl (5-((5-chloro-1H-pyrrolo[2,3-b]pyridin-3-yl)methyl)pyridin-2-yl)((6-(trifluoromethyl)pyridin-3-yl)methyl)carbamate (PXDP)

### Example 4-1. Synthesis of intermediate (5-((5-chloro-1H-pyrrolo[2,3-b]pyridin-3-yl)methyl)pyridin-2-yl)((6-(trifluoromethyl)pyridin-3-yl)methyl)carbamic chloride

(5-((5-chloro-1H-pyrrolo[2,3-b]pyridin-3-yl)methyl)-N-((6-(trifluoromethyl)pyridin-3-yl)methyl)pyridin-2-amine (500 mg, 1.19 mmol) was suspended in anhydrous dichloromethane (10 mL), followed by the addition of pyridine (141.9 mg, 1.79 mmol), and the mixture was stirred under a nitrogen atmosphere at 0°C to 5°C. Triphosgene (213.0 mg, 0.71 mmol) was added, and the mixture was stirred at room temperature for at least 1 hour. The reaction mixture was concentrated under reduced pressure, and the resulting residue was purified by silica gel column chromatography (5% MC:MeOH) to afford a light yellow solid (235.6 mg, 41.0%).

### Example 4-2. Synthesis of 2-(4-methylpiperazin-1-yl)ethyl (5-((5-chloro-1H-pyrrolo[2,3-b]pyridin-3-yl)methyl)pyridin-2-yl)((6-(trifluoromethyl)pyridin-3-yl)methyl)carbamate

2-(4-methylpiperazin-1-yl)ethan-1-ol (66.0 mg, 0.45 mmol) was added to anhydrous dichloromethane (4.0 mL), followed by the addition of 60% sodium hydride (24.9 mg, 0.62 mmol) under a nitrogen atmosphere at 0°C to 5°C. The mixture was then stirred. (5-((5-chloro-1H-pyrrolo[2,3-b]pyridin-3-yl)methyl)pyridin-2-yl)((6-(trifluoromethyl)pyridin-3-yl)methyl)carbamic chloride (200 mg, 0.41 mmol) was added, and the mixture was stirred at room temperature for at least 1 hour. The reaction mixture was concentrated under reduced pressure, and the resulting residue was purified by silica gel column chromatography (15% MC:MeOH) to afford a yellow solid (76.4 mg, 31.2%).

Chemical Formula: C₂₈H₂₉ClF₃N₇O₂

Exact Mass: 587.20
LC/MS: [M+H] 588.21
¹H-NMR(DMSO-d6): δ 11.74(1H), 8.73(1H), 8.38(1H), 8.17(1H), 8.04(1H), 7.98-7.96(1H), 7.83-7.81(1H), 7.71-7.47(2H), 7.47(1H), 5.19(2H), 4.20-4.17(2H), 4.03(2H), 2.47-2.44(2H), 2.27-2.16(8H), 2.07(3H)

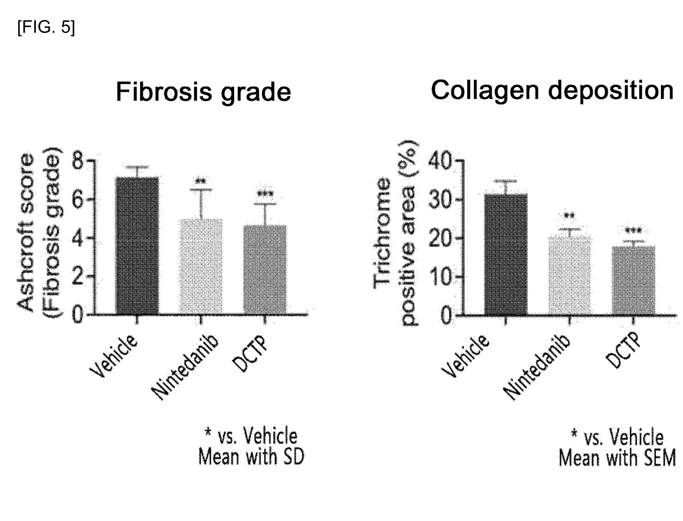

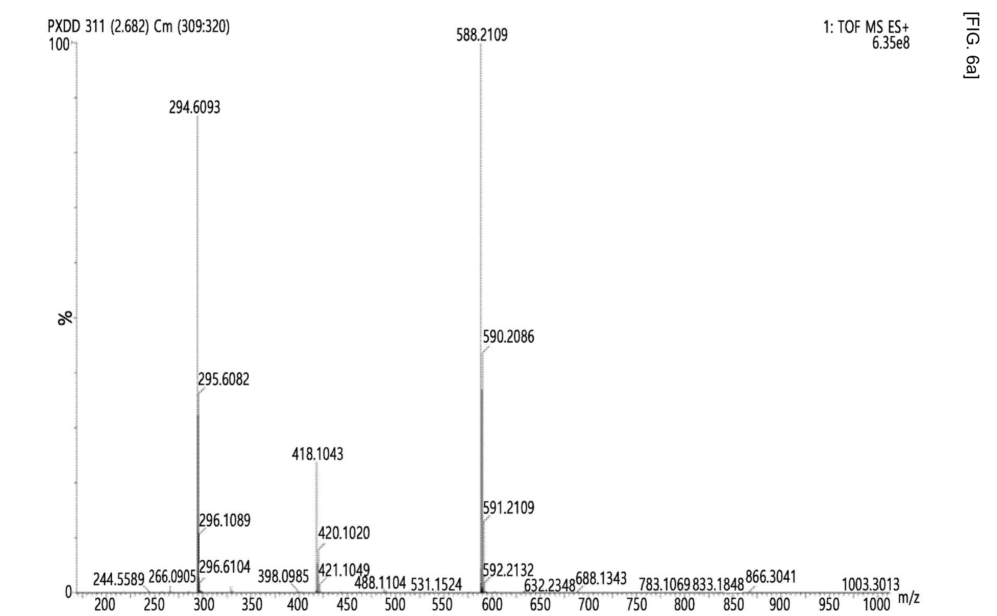

MS and NMR data are shown in FIG. 6, and the obtained compound, 2-(4-methylpiperazin-1-yl)ethyl (5-((5-chloro-1H-pyrrolo[2,3-b]pyridin-3-yl)methyl)pyridin-2-yl)((6-(trifluoromethyl)pyridin-3-yl)methyl)carbamate (hereinafter referred to as "PXDP"), was used in Examples 4 and 5 below.

### Example 5. Confirmation of inhibitory effect on idiopathic pulmonary fibrosis induced by bleomycin

Bleomycin sulfate was administered at a dose of 1.5 U/kg to six-week-old male C57BL/6 mice, and the mice were observed for pulmonary fibrosis (10 mice per group). Each drug was administered starting 14 days after the administration of bleomycin. Nintedanib and PXDP were each orally administered at a dose of 60 mg/kg once daily for two weeks (a total of 13 administrations), and the group treated with the combination was orally administered nintedanib (60 mg/kg) together with PXDP (60 mg/kg) once daily for two weeks (a total of 13 administrations).

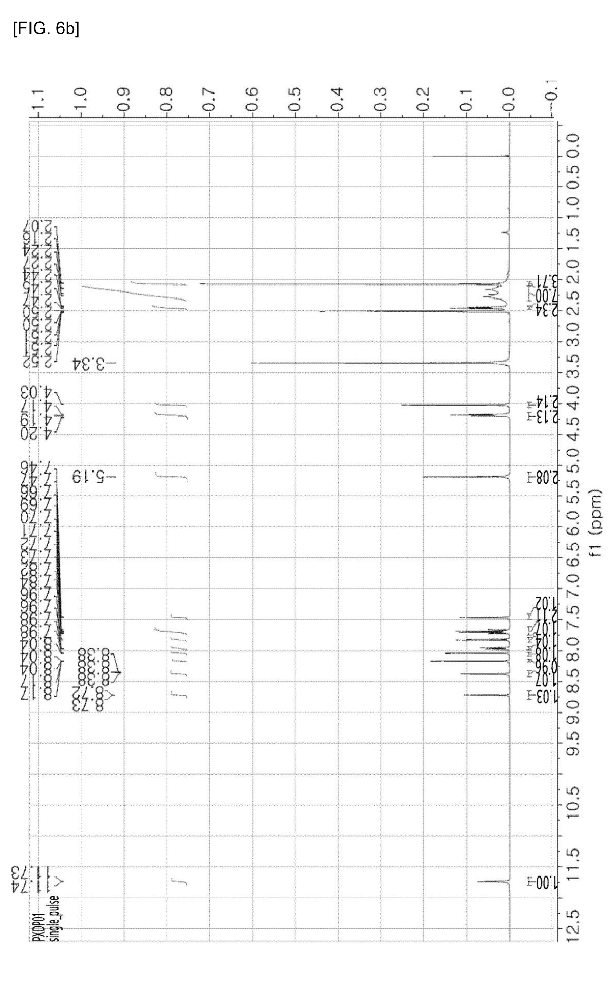

The results of Micro-CT imaging performed 4 weeks after bleomycin administration are shown in FIG. 7.

In the untreated group, progression of fibrosis was confirmed 4 weeks after bleomycin administration. In addition, a reduction in the degree of fibrosis was confirmed in groups treated with nintedanib, which is a known antifibrotic agent, and PXDP. In particular, the group treated with the combination of nintedanib and DCTP exhibited a greater antifibrotic effect.

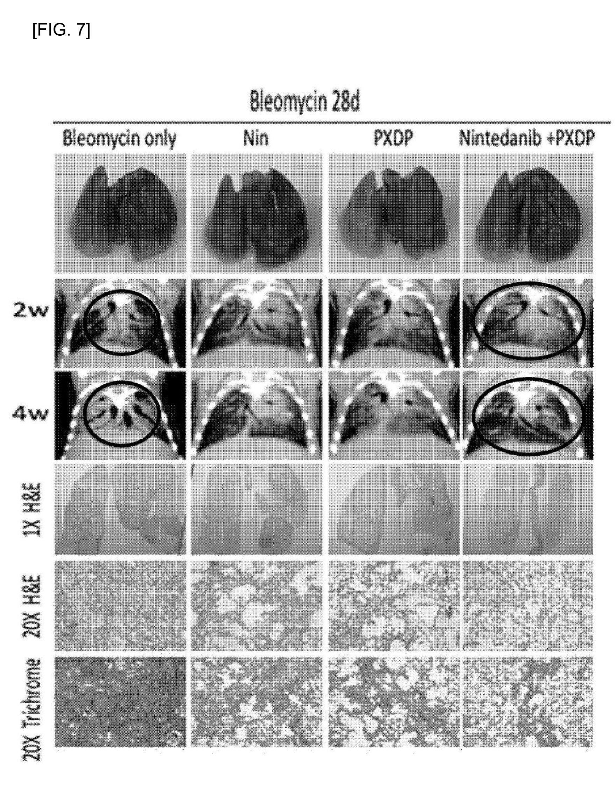

The results of H&E and Masson's Trichrome staining performed to evaluate the degree of inflammation and fibrosis are shown in FIG. 8.

Compared to the untreated group, the group treated with nintedanib and the group treated with PXDP showed alleviation of pulmonary fibrosis and inflammation. In particular, the group treated with the combination of nintedanib and PXDP showed a greater reduction in the degree of fibrosis and inflammation.

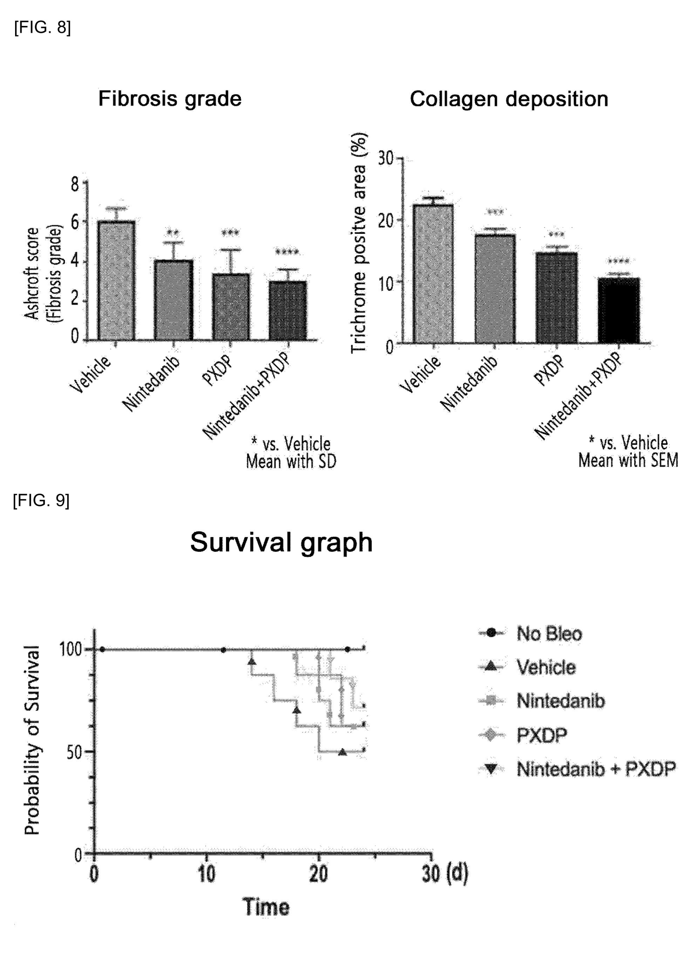

In addition, the mouse survival rate was compared in FIG. 9.

As a result of examining the survival rate for each treatment group, it was found that, compared to the untreated group, the survival rate increased in the group treated with nintedanib and the group treated with PXDP, and in particular, the group treated with the combination of nintedanib and PXDP showed a marked increase in survival rate.

### Example 6. Confirmation of inhibitory effect on radiation-induced pulmonary fibrosis

One hour prior to stereotactic body radiation therapy (SBRT; 90 Gy, 4 mm collimator), PXDP and nintedanib, an FDA-approved drug known to inhibit pulmonary fibrosis, were orally administered to seven-week-old male C57BL/6 mice. The degree of pulmonary fibrosis and the inhibitory effect on radiation-induced lung injury were observed two weeks after radiation exposure. The results of Micro-CT imaging performed 2 weeks after radiation exposure are shown in FIG. 10.

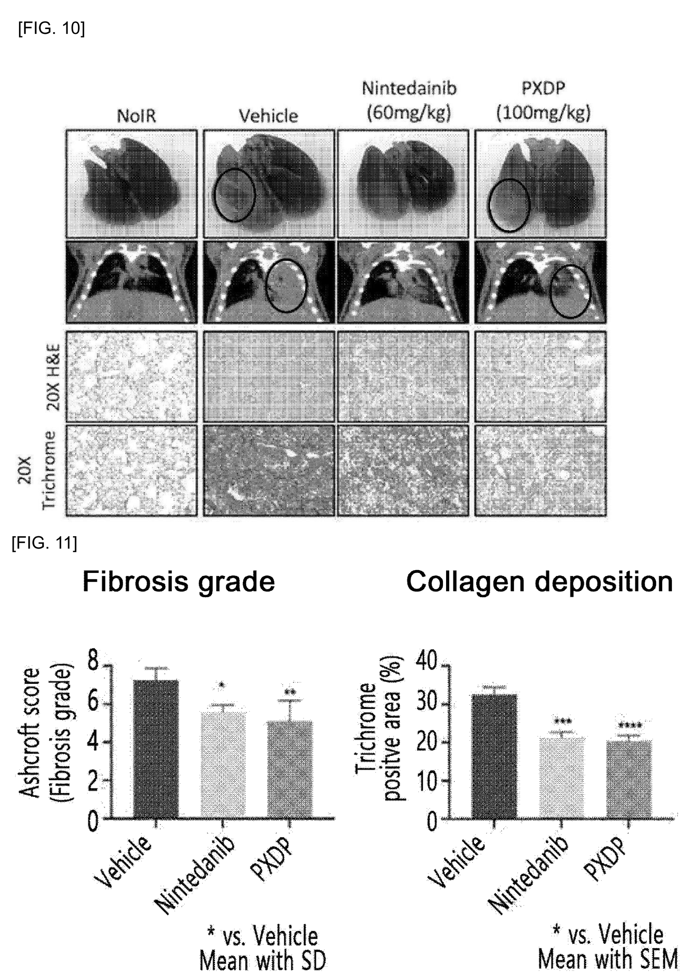

The PXDP drug was administered at a dose of 100 mg/kg, and nintedanib was administered once daily (o.d.) at a dose of 60 mg/kg. To analyze the degree of inflammatory response and the degree of fibrosis 2 weeks after radiation exposure, H&E staining and Masson's Trichrome staining were performed, and the results are shown in FIG. 11.

It was found that, in the untreated group, progression of fibrosis was observed two weeks after radiation exposure, whereas in the group treated with PXDP and the group treated with nintedanib, a known antifibrotic agent, the results of Micro-CT imaging confirmed a reduction in the degree of fibrosis. In particular, it was found that the effect of PXPD was superior to that of nintedanib.

Additionally, the results of H&E and Masson's Trichrome staining that were performed to confirm the degree of inflammation and the degree of fibrosis confirmed that, compared to the untreated group, both the nintedanib-treated group and the PXPD-treated group exhibited alleviation of fibrosis and inflammation in the lungs.

From the above description, those skilled in the art to which the present invention pertains would understand that the present invention can be implemented in other specific forms without changing its technical concept or essential features. Therefore, the embodiments described above should be construed as being exemplified and not limiting the present invention. The scope of the present invention should be interpreted to include all modifications or modified forms derived from the meaning and scope of the claims as set forth below, as well as equivalent concepts, rather than from the detailed description above.

## Claims

1. A compound represented by following Formula 1, or a pharmaceutically acceptable salt or solvate thereof: 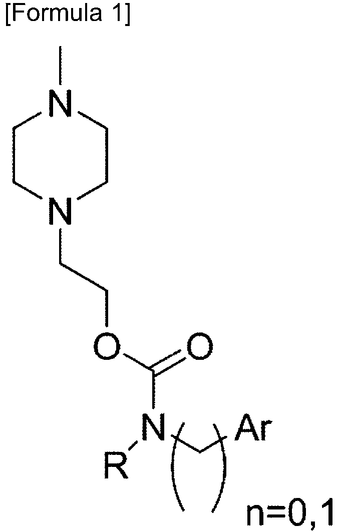
wherein in Formula 1 above, Ar is aryl or heteroaryl, substituted with trifluoromethyl (CF3) or halogen,
and R is represented by following Formula 2 or Formula 3:

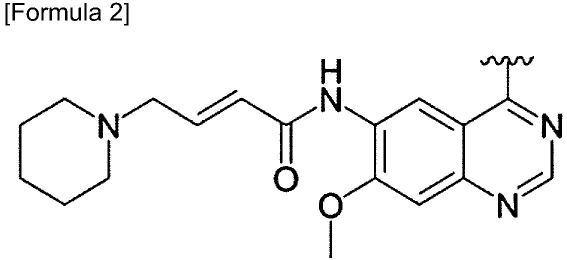

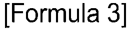

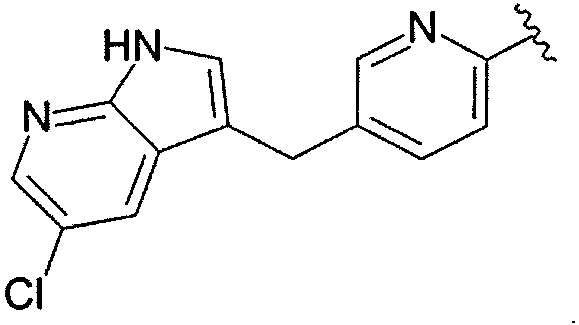

2. The compound of claim 1, wherein Ar in Formula 1 is

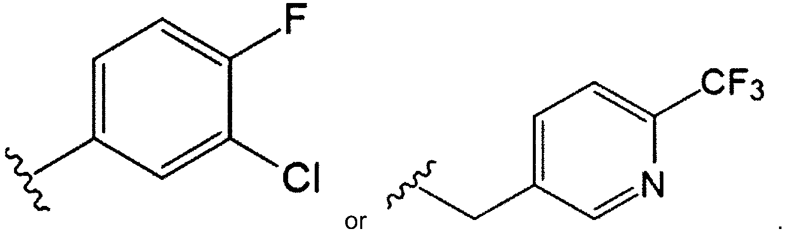

3. The compound of claim 1, wherein the compound is represented by Formula 4 or Formula 5:

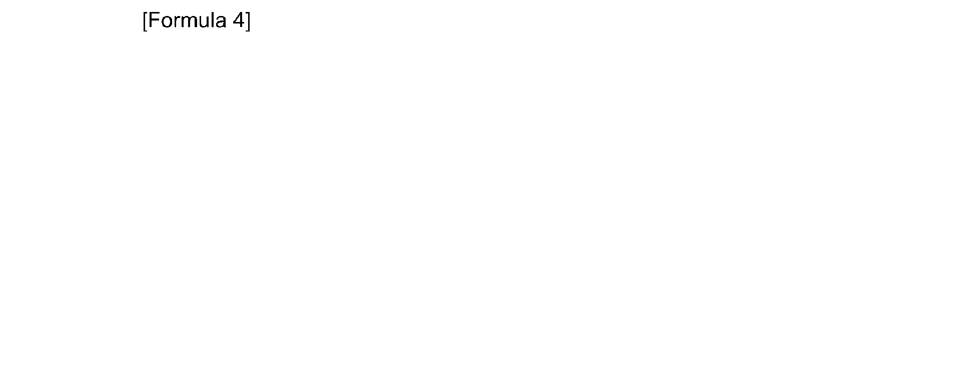

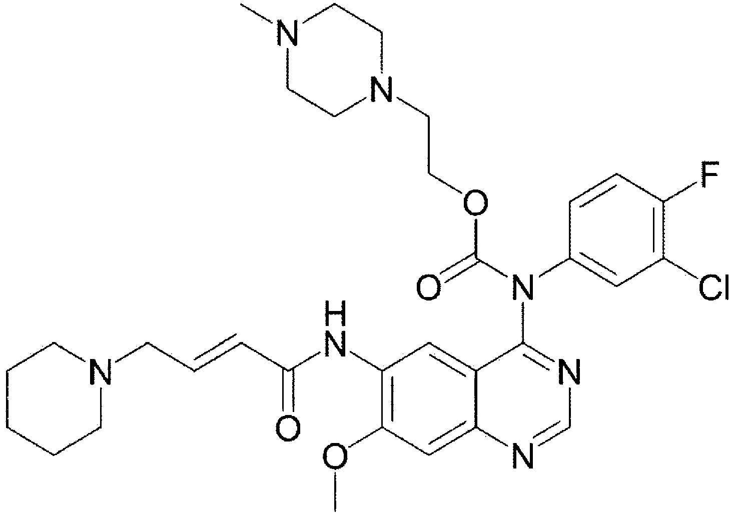

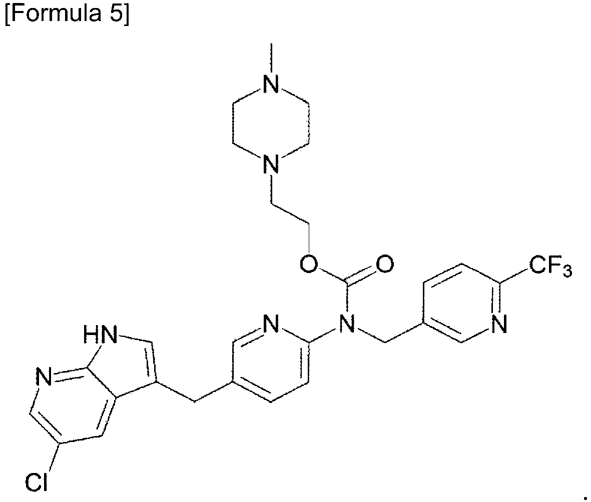

4. A composition comprising a compound represented by following Formula 1 or a pharmaceutically acceptable salt or solvate thereof, and a pharmaceutically acceptable carrier: 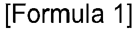 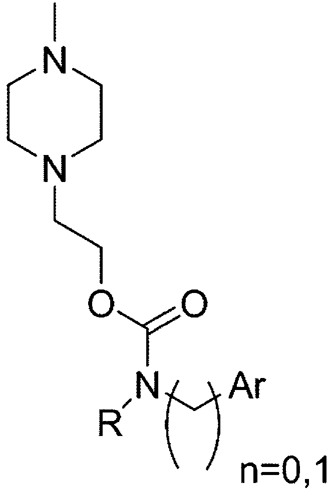
wherein in Formula 1 above, Ar is aryl or heteroaryl, substituted with trifluoromethyl (CF3) or halogen,
and R is represented by following Formula 2 or Formula 3:

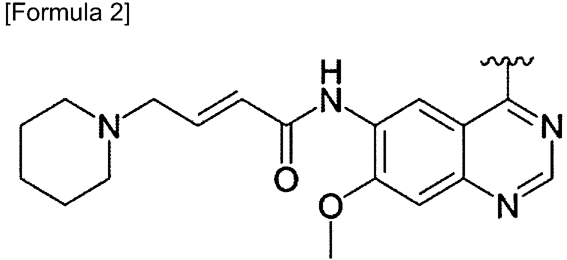

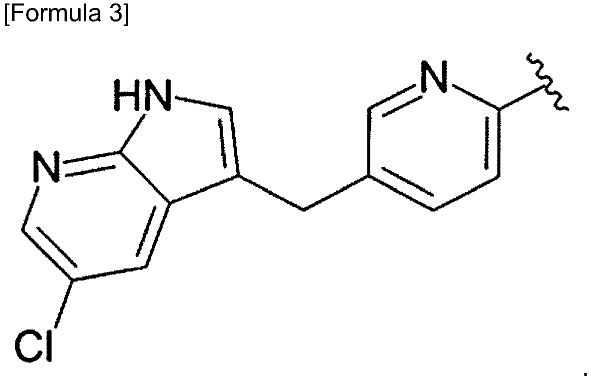

5. A pharmaceutical composition for preventing or treating pulmonary fibrosis, comprising a compound represented by following Formula 1 or a pharmaceutically acceptable salt or solvate thereof: 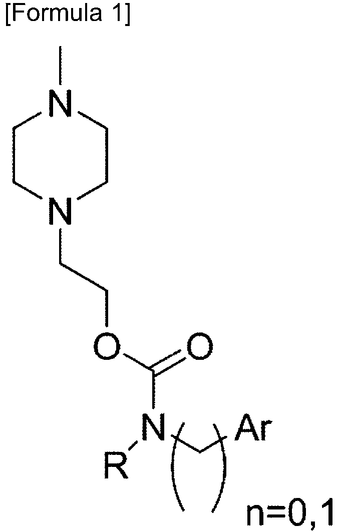
wherein in Formula 1 above, Ar is aryl or heteroaryl, substituted with trifluoromethyl (CF3) or halogen,
and R is represented by following Formula 2 or Formula 3:

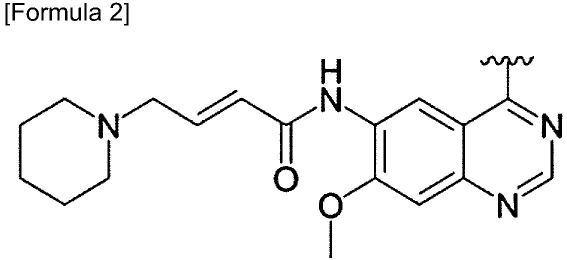

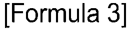

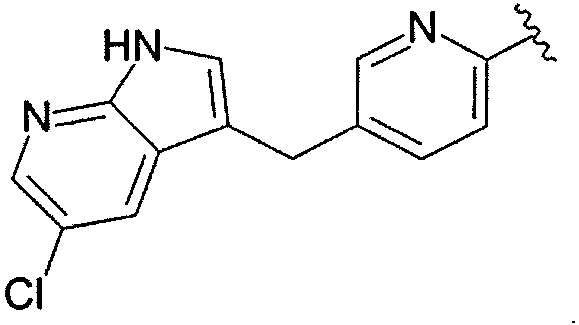

6. The pharmaceutical composition of claim 5, wherein the pulmonary fibrosis is selected from pulmonary fibrosis caused by radiation exposure and idiopathic pulmonary fibrosis.

7. The pharmaceutical composition of claim 5, wherein the pharmaceutical composition is co-administered with a pharmaceutical composition comprising nintedanib as an active ingredient.

8. A method for preventing or treating pulmonary fibrosis, comprising administering the pharmaceutical composition of any one of claims 5 to 7 to a subject.

9. A food composition for preventing or improving pulmonary fibrosis, comprising a compound represented by following Formula 1 or a food-acceptable salt or solvate thereof: 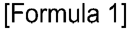 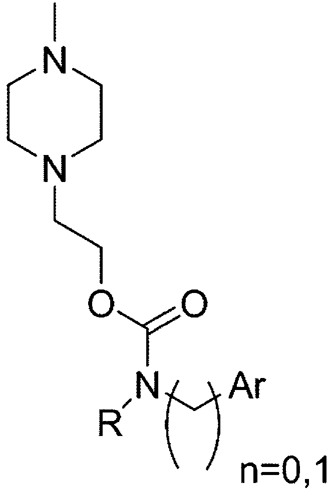
wherein in Formula 1 above, Ar is aryl or heteroaryl, substituted with trifluoromethyl (CF3) or halogen,
and R is represented by following Formula 2 or Formula 3:
